# NEW EUROPEAN PATENT SPECIFICATION

(11) **EP 1 587 924 B2**
(45) Date of publication and mention of the opposition decision: **18.01.2012**
(45) Mention of the grant of the patent: 02.01.2008
(21) Application number: 04703064.8
(22) Date of filing: 16.01.2004
(51) Int. Cl.: C12N 15/00, C12N 15/09, C12N 15/63, C12N 15/70, C12N 15/74, C12N 15/87, C12N 15/85, C12N 15/10, C12N 15/75, C12R 1/125

(54) **METHODS FOR SITE-DIRECTED MUTAGENESIS AND TARGETED RANDOMIZATION**
VERFAHREN ZUR STELLENGERICHTETEN MUTAGENESE UND GEZIELTEN RANDOMISIERUNG
PROCEDES DE MUTAGENESE DIRIGES SUR SITE ET D'ECHANTILLONNAGE ALEATOIRE CIBLE

(30) Priority: 16.01.2003 US 440792 P
(43) Date of publication of application: 26.10.2005
(73) Proprietor: GENENCOR INTERNATIONAL, INC., Palo Alto, CA 94117 (US)
(72) Inventor: LEEFLANG, Chris, NL-2592SZ The Hague (NL); VAN DER KLEIJ, Wilhelmus, A., H., NL-2548CZ The Hague (NL)
(74) Representative: Kiddle, Simon John
(86) International application number: PCT/US2004/001334
(87) International publication number: WO 2004/064744

(56) References cited:
- US-A- 5 418 873
- US-A- 5 776 746
- US-A1- 2003 162 209
- SHAFIKHANI ET AL: "Generation of large libraries of random mutants in Bacillus subtilis by PCR-based plasmid multimerization" BIOTECHNIQUES, INFORMA LIFE SCIENCES PUBLISHING, WESTBOROUGH, MA, US, vol. 23, no. 2, 1997, pages 304-310, XP002094373 ISSN: 0736-6205
- MELNIKOV ALEXANDRE ET AL: "Random mutagenesis by recombinational capture of PCR products in Bacillus subtilis and Acinetobacter calcoaceticus" NUCLEIC ACIDS RESEARCH, vol. 27, no. 4, 15 February 1999 (1999-02-15), pages 1056-1062, XP002372118 ISSN: 0305-1048
- PARIKH ASIT ET AL: "RANDOM MUTAGENESIS BY WHOLE-PLASMID PCR AMPLIFICATION" BIOTECHNIQUES, INFORMA LIFE SCIENCES PUBLISHING, WESTBOROUGH, MA, US, vol. 24, no. 3, March 1998 (1998-03), pages 428-431, XP001109654 ISSN: 0736-6205
- BEIER ET AL: 'Conversion of the maltogenic alpha-amylase Novamyl into a CGTase.' PROTEIN ENGINEERING vol. 13(7), 01 January 2000, pages 509 - 513
- WONG ET AL: 'Engineering and production of streptokinase and their in a Bacillus subtilis expression-secretion system' APPL.ENVIRON.MICROBIOL. 01 January 1994, pages 517 - 523

### SEQUENCE LISTING

<110> Genencor International, Inc.
<120> Methods for Site-Detected Mutagenesis and Targeted Randomization
<130> SJK/FP6316905
<140> EP 04703064.8
   <141> 2004-01-16
<150> PCT/US04/01334
   <151> 2004-01-16
<150> US 60/440,782
   <151> 2003-01-16
<160> 6
<170> FastSEQ for Windows Version 4.0
<210> 1
   <211> 275
   <212> PRT
   <213> Bacillus amyloliquefaciens
<400> 1
<210> 2
   <211> 275
   <212> PRT
   <213> Bacillus subtilis
<400> 2
   Ala Gln Ser Val Pro Tyr Gly Ile Ser Gln Ile Lys Ala Pro Ala Leu
   1               5                   10                  15
   His Ser Gln Gly Tyr Thr Gly Ser Asn Val Lys Val Ala Val Ile Asp
               20                  25                  30
   Ser Gly Ile Asp Ser Ser His Pro Asp Leu Asn Val Arg Gly Gly Ala
           35                  40                  45
   Ser Phe Val Pro Ser Glu Thr Asn Pro Tyr Gln Asp Gly Ser Ser His
       50                  55                  60
   Gly Thr His Val Ala Gly Thr Ile Ala Ala Leu Asn Asn Ser Ile Gly
   65                  70                  75                  80
   Val Leu Gly Val Ser Pro Ser Ala Ser Leu Tyr Ala Val Lys Val Leu
                   85                  90                  95
   Asp Ser Thr Gly Ser Gly Gln Tyr Ser Trp Ile Ile Asn Gly Ile Glu
               100                 105                 110
   Trp Ala Ile Ser Asn Asn Met Asp Val Ile Asn Met Ser Leu Gly Gly
           115                 120                 125
   Pro Thr Gly Ser Thr Ala Leu Lys Thr Val Val Asp Lys Ala Val Ser
       130                 135                 140
   Ser Gly Ile Val Val Ala Ala Ala Ala Gly Asn Glu Gly Ser Ser Gly
   145                 150                 155                 160
   Ser Thr Ser Thr Val Gly Tyr Pro Ala Lys Tyr Pro Ser Thr Ile Ala
                   165                 170                 175
   Val Gly Ala Val Asn Ser Ser Asn Gln Arg Ala Ser Phe Ser Ser Ala
               180                 185                 190
   Gly Ser Glu Leu Asp Val Met Ala Pro Gly Val Ser Ile Gln Ser Thr
           195                 200                 205
   Leu Pro Gly Gly Thr Tyr Gly Ala Tyr Asn Gly Thr Ser Met Ala Thr
       210                 215                 220
   Pro His Val Ala Gly Ala Ala Ala Leu Ile Leu Ser Lys His Pro Thr
   225                 230                 235                 240
   Trp Thr Asn Ala Gln Val Arg Asp Arg Leu Glu Ser Thr Ala Thr Tyr
                   245                 250                 255
   Leu Gly Asn Ser Phe Tyr Tyr Gly Lys Gly Leu Ile Asn Val Gln Ala
               260                 265                 270
   Ala Ala Gln
           275
<210> 3
   <211> 274
   <212> PRT
   <213> Bacillus licheniformis
<400> 3
<210> 4
   <211> 269
   <212> PRT
   <213> Bacillus lentus
<400> 4
   Ala Gln Ser Val Pro Trp Gly Ile Ser Arg Val Gln Ala Pro Ala Ala
   1 5 10 15
   His Asn Arg Gly Leu Thr Gly Ser Gly Val Lys Val Ala Val Leu Asp
   20 25 30
   Thr Gly Ile Ser Thr His Pro Asp Leu Asn Ile Arg Gly Gly Ala Ser
   35 40 45
   Phe Val Pro Gly Glu Pro Ser Thr Gln Asp Gly Asn Gly His Gly Thr
   50 55 60
   His Val Ala Gly Thr Ile Ala Ala Leu Asn Asn Ser Ile Gly Val Leu
   65 70 75 80
   Gly Val Ala Pro Ser Ala Glu Leu Tyr Ala Val Lys Val Leu Gly Ala
   85 90 95
   Ser Gly Ser Gly Ser Val Ser Ser Ile Ala Gln Gly Leu Glu Trp Ala
   100 105 110
   Gly Asn Asn Gly Met His Val Ala Asn Leu Ser Leu Gly Ser Pro Ser
   115 120 125
   Pro Ser Ala Thr Leu Glu Gln Ala Val Asn Ser Ala Thr Ser Arg Gly
   130 135 140
   Val Leu Val Val Ala Ala Ser Gly Asn Ser Gly Ala Gly Ser Ile Ser
   145 150 155 160
   Tyr Pro Ala Arg Tyr Ala Asn Ala Met Ala Val Gly Ala Thr Asp Gln
   165 170 175
   Asn Asn Asn Arg Ala Ser Phe Ser Gln Tyr Gly Ala Gly Leu Asp Ile
   180 185 190
   Val Ala Pro Gly Val Asn Val Gln Ser Thr Tyr Pro Gly Ser Thr Tyr
   195 200 205
   Ala Ser Leu Asn Gly Thr Ser Met Ala Thr Pro His Val Ala Gly Ala
   210 215 220
   Ala Ala Leu Val Lys Gln Lys Asn Pro Ser Trp Ser Asn Val Gln Ile
   225 230 235 240
   Arg Asn His Leu Lys Asn Thr Ala Thr Ser Leu Gly Ser Thr Asn Leu
   245 250 255
   Tyr Gly Ser Gly Leu Val Asn Ala Glu Ala Ala Thr Arg
   260 265
<210> 5
   <211> 27
   <212> DNA
   <213> Artificial Sequence
<220>
   <223> primer
<400> 5
   gtgtgtgggc ccatcagtct gacgacc 27
<210> 6
   <211> 27
   <212> DNA
   <213> Artificial Sequence
<220>
   <223> primer
<400> 6
   gtgtgtgggc cctattcgga tattgag 27

## Description

### FIELD OF THE INVENTION

The present invention provides methods and compositions for the direct transformation of engineered plasmids and controlled randomized plasmid libraries in *Bacillus.* In particular, the present invention provides means that avoid the need for the use of intermediate hosts, such as *E*. *coli* for the development of *Bacillus* strains suitable for the production of proteins.

### BACKGROUND OF THE INVENTION

*Bacillus* species *(e.g., B. subtilis)* are among the preferred screening hosts for many protein evolution and other projects that involve developments in protein production. However, direct transformation of DNA libraries, such as site-saturation libraries and targeted randomization in these organisms is highly inefficient using methods known in the art. Indeed, due to the limited availability of cloning methods that work well in Bacillus, the modification and/or improvement of expressed proteins has proven difficult. Thus, as discussed below, libraries are typically first made in *E. coli* and then introduced into *Bacillus.* This indirect approach presents numerous limitations, including the need for longer protein engineering/development times, the inability to use desired plasmid systems due to toxicity demonstrated by *E. coli,* library bias, and the inability to make high throughput screening a robust process.

As indicated above, widely used methods for altering the plasmids of *Bacillus* involve building plasmid constructs and first transforming them into *E. coli.* Subsequently, the plasmids (typically, replicating plasmids) are isolated from *E. coli* and transformed into *Bacillus.* Widespread use of this method can be attributed, at least in part, to the belief among those in the art that *E. coli* is easier to transform than *Bacillus.* This is partially due to the limited efficiency of *in vitro* ligation of plasmids that results in nicked products and monomeric DNA being capable of transforming *E. coli,* but which do not effectively transform *Bacillus.*

It has been observed, that multimers of replicating plasmids are significantly more efficient at transforming Bacillus as compared to monomers (*See e.g.,* Mottes et al., (1979) Molec. Gen. Genet., 174:281-286 [1979]). However, traditional methods for plasmid mutagenesis generally do not produce plasmid multimers. Thus, typical mutagenesis products cannot be efficiently transformed into *Bacillus.* Multimers of plasmids can be formed in vitro by ligation of linear plasmids at very high DNA concentrations (*See*, Mottes *et al., supra*). Multimers can also be formed via a PCR-like reaction starting from two overlapping plasmid fragments as template (Shafikhani et al., BioTechn., 3:304-310 [1997]). However, this process is rather mutagenic given the long extension cycles that are required.

An alternative method that allows the generation of plasmid libraries in *Bacillus* is plasmid marker rescue *(See,* Contente and Dubnau, Plasmid 2:555-571 [1979]). However, a disadvantage of this method is the requirement for a resident plasmid in the competent strain and the prolonged coexistence of several plasmids in the transformed cells.

In addition to the disadvantages listed above, the larger the sequence, the more difficult it is to insert and obtain replication. Additionally, there are sequences that will not replicate in *E*. *coli,* resulting in a loss of diversity in the DNA library being built. Furthermore, the high copy number of some plasmids/vectors is often deleterious to *E. coli.*

Alternatives to replicating plasmids are sometimes used, including integrating plasmids and vectors. Integrating vectors do not contain an origin of replication and therefore require insertion into the host chromosome to be stably maintained. Integration occurs via a Campbell-type recombination event that results in a duplication of the cloned region at either end of the inserted (now linear) vector. Depending on the position of the integration, genes may be disrupted resulting in poor transformation efficiency.

Despite much work in the area, the prior art methods fail to reproducibly provide methods suitable for mutagenesis of replicating plasmids in *Bacillus* and for the easy generation of large libraries in *Bacillus* and other host cells. Thus, there is a need for a *Bacillus* transformation method that is relatively straightforward, efficient and reproducible. In particular, a method is needed that permits the efficient transformation of *Bacillus,* without requiring intervening steps involving the use of additional microorganisms, such as *E*. *coli.* Indeed, there remains a need for methods that eliminate the need to utilize *E*. *coli* and directly introduce libraries into the *Bacillus* species of interest, in order to produce the protein(s) of interest.

### SUMMARY OF THE INVENTION

The present invention provides methods for the construction and direct transformation of engineered plasmids and controlled randomized plasmid libraries in *Bacillus.* In particular, the present invention provides means that avoid the need for the use of intermediate hosts, such as *E*. *coli* for the development of *Bacillus* strains suitable for the production of proteins. In particular, in the present invention, methods are provided which utilize fusion polymerase chain reaction techniques for the *in vitro* generation of modified sequences that can effectively transform Bacillus.

In one embodiment, a method for direct transformation of a Bacillus host cell is provided comprising the steps:
(a) generating partially overlapping intermediate fragments by polymerase chain reaction, said partially overlapping intermediate fragments further comprising a first intermediate fragment and a second intermediate fragment,
   wherein said first intermediate fragment is generated by polymerase chain reaction using a forward mutagenic primer and a reverse digestion site primer, and a plasmid template which is an expression vector capable of replicating within the Bacillus host cell, and
   wherein said second intermediate fragment is generated by polymerase chain reaction using a reverse mutagenic primer and a forward digestion site primer and the plasmid template,
   such that said first and second intermediate fragments each comprise at least one mutated codon of interest, a flanking nucleotide sequence and a digestion site
(b) joining ends of said intermediate fragments to produce a linear product by fusion polymerase chain reaction;
(c) digesting the linear product with a restriction enzyme to create cohesive ends on the linear product;
(d) ligating the cohesive ends of the linear product to create a circular product which is an expression vector capable of replicating within the Bacillus host cell; and
(e) incubating said Bacillus host cell with said circular product.

In another embodiment, the digestion site is an *Apal* digestion site.

In another embodiment, the forward digestion site primers comprises a polynucleotide sequence GTGTGTGGGCCCATCAGTCTGACGACC.

In another embodiment, the reverse digestion site primers comprises the polynucleotide sequence GTGTGTGGGCCCTATTCGGATATTGAG.

In another embodiment, a method for constructing a vector for direct transformation of a Bacillus host cell is provided, the method comprising the steps:
(a) generating partially overlapping intermediate fragments by polymerase chain reaction, said partially overlapping intermediate fragments further comprising a first intermediate fragment and a second intermediate fragment,
   wherein said first intermediate fragment is generated by polymerase chain reaction using a forward mutagenic primer and a reverse digestion site primer, and a plasmid template which is an expression vector capable of replicating within the Bacillus host cell, and
   wherein said second intermediate fragment is generated by polymerase chain reaction using a reverse mutagenic primer and a forward digestion site primer and the plasmid template,
   and wherein said forward and reverse mutagenic primers have an overlapping portion upstream and downstream of a codon of interest,
   such that said first and second intermediate fragments each comprise at least one mutated codon of interest, a flanking nucleotide sequence and a digestion site;
(b) joining ends of said intermediate fragments to produce a linear product by fusion polymerase chain reaction;
(c) digesting the linear product with a restriction enzyme to create cohesive ends on the linear product; and
(d) ligating of the cohesive ends of the linear product to create a circular polynucleotide sequence which is an expression vector capable of replicating within the Bacillus host cell.

### BRIEF DESCRIPTION OF THE DRAWINGS

Fig. 1 depicts a pVS08 Bacillus subtilis expression vector.
Fig. 2 depicts the orientation of the forward ApaI primer, the reverse ApaI primer, the reverse mutagenic primer, and the forward mutagenic primer.

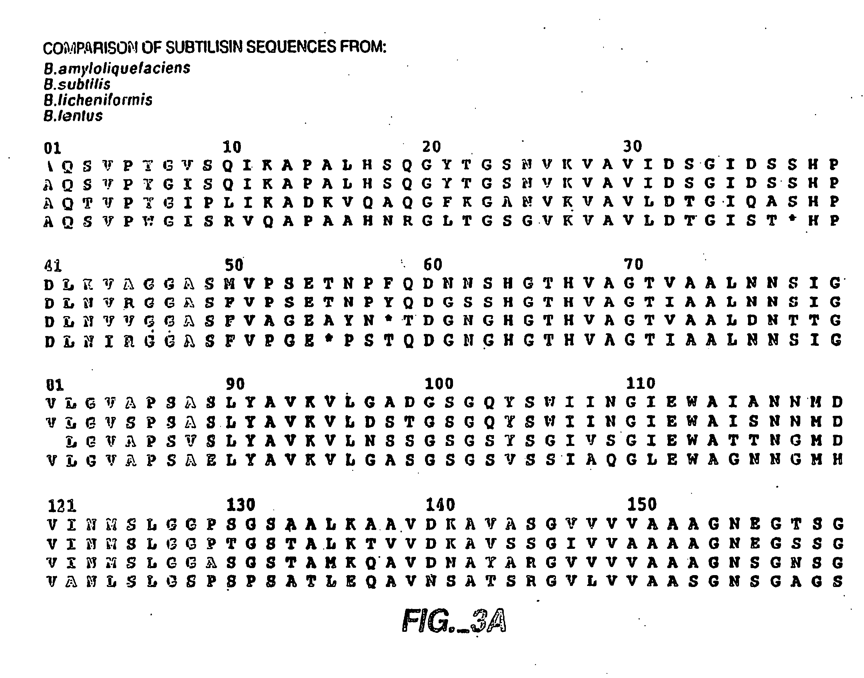

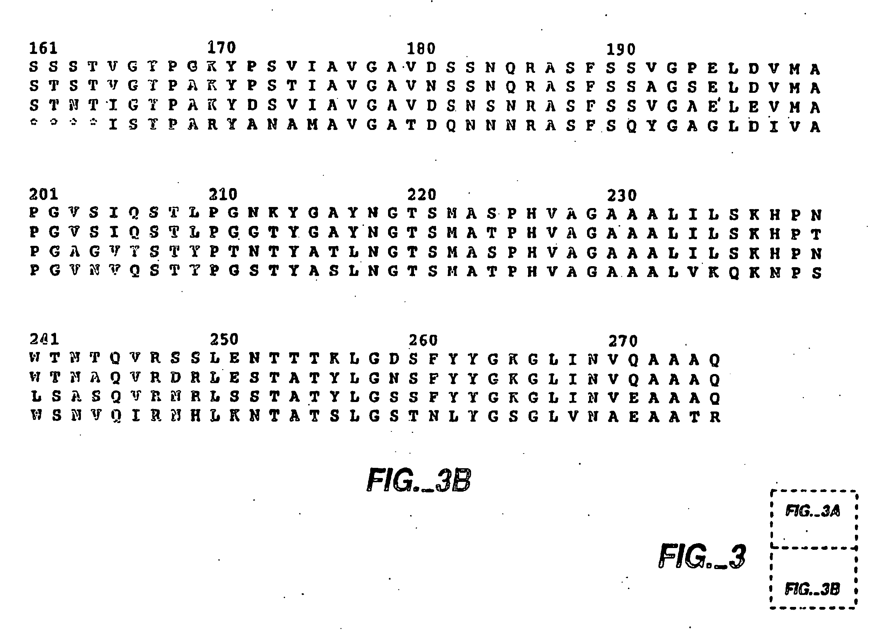

Figs. 3A and 3B depict the amino acid sequence of four subtilisins. The top line represents the amino acid sequence of subtilisin from *Bacillus amyloliquefaciens* subtilisin (also sometimes referred to as subtilisin BPN') (SEQ ID NO:1). The second line depicts the amino acid sequence of subtilisin from *Bacillus subtilis* (SEQ ID NO:2). The third line depicts the amino acid sequence of subtilisin from *B. licheniformis* (SEQ ID NO:3). The fourth line depicts the amino acid sequence of subtilisin from *Bacillus lentus* (also referred to as subtilisin 309 in PCT WO89/06276) (SEQ ID NO:4). The symbol * denotes the absence of specific amino acid residues as compared to subtilisin BPN'.
Fig. 4 depicts the polynucleotide sequence of the forward *Apa*I primer (SEQ ID No.: 5).
Fig. 5 depicts the polynucleotide sequence of the reverse *Apa*I primer (SEQ ID NO.:6)
Fig. 6 depicts a schematic overview of the fusion polymerase chain reaction (third PCR) in which two intermediate fragments both comprising the mutated codon of interest are fused using the forward and reverse restriction (*Apa*I) primer sets.
Fig. 7 depicts a schematic overview of the fused linear product of conjoined intermediate fragments.

### DESCRIPTION OF THE INVENTION

The present invention provides methods for the construction and direct transformation of engineered plasmids and controlled randomized plasmid libraries in *Bacillus.* In particular, the present invention provides means that avoid the need for the use of intermediate hosts, such as *E. coli* for the development of *Bacillus* strains suitable for the production of proteins. In particular, in the present invention, methods are provided which utilize fusion polymerase chain reactions ("PCR") for the *in vitro* generation of modified sequences that can effectively transform *Bacillus.*

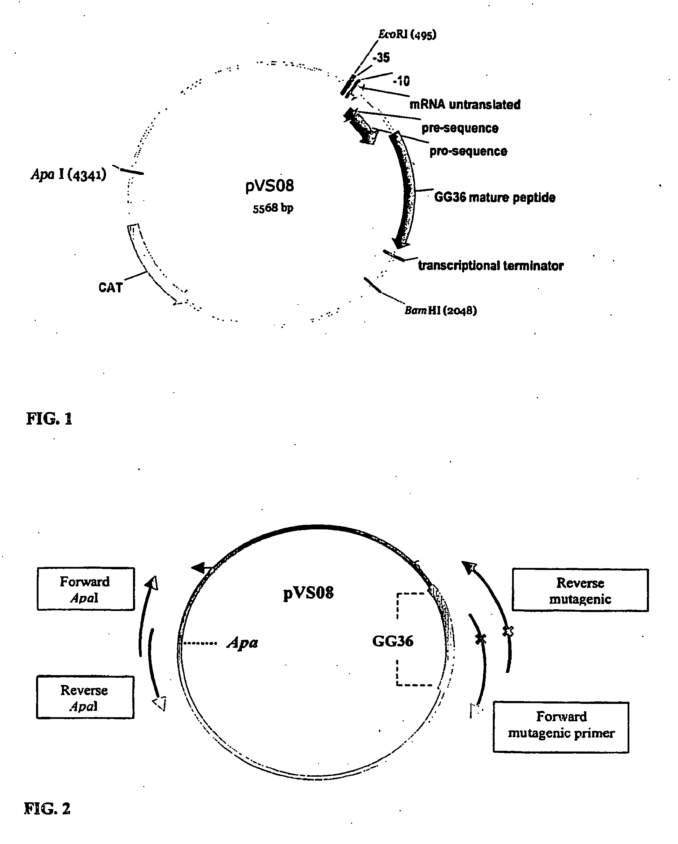

The direct *Bacillus* transformation methods of the present invention involve construction of DNA libraries and/or mutants using fusion PCR techniques (Vallejo, A.N. (1995), PCR Primer: a Laboratory Manual [Dieffenbach, C.W., Dvekster, G.S., eds.) pp. 603-612, Cold Spring Harbour Laboratory Press. Cold Spring Harbour, New York) are used. PCR techniques are used to generate the DNA fragments for subsequent ligation. This PCR technique is used to generate the fragments and complete length of DNA desired for transformation and facilitates direct transformation of the DNA into *Bacillus (*e.g*., B. subtilis).* During the development of the present invention, the pVS08 plasmid (Fig. 1, this plasmid is similar to pVS02, but has a shorter aprE promoter) and the *Apa*I restriction site were utilized. However, it is not intended that the present invention be limited to any particular plasmid or restriction site, as it is contemplated that various plasmids and restriction sites will find use in the present invention.

Optionally, the *Dpn*I enzyme is used to degrade template DNA that could otherwise lead to the transformation of host cells with un-mutated plasmid. *Dpn*I is known to cleave methylated DNA strands. Methylated template can be generated by isolating template from any organism that methylates its DNA, for example *dam*⁺ strains of *E. coli.* Alternatively, template DNA can be methylated *in vitro* using *dam* methylases (*See e.g.,* Kim and Maas, Biotechn., 28:196-198 [2000]).

To generate mutants or libraries by fusion PCR, three PCR reactions are carried out. Two PCR reactions are performed to generate partially overlapping intermediate fragments. A third PCR reaction is carried out to fuse the intermediate fragments as more fully described in this application. The method for construction the library or mutant variants includes constructing a first set of primers around a desired restriction site (restriction site primer), a forward and reverse restriction primer and a second set of primers around, e.g., upstream and downstream of the codon of interest (the mutagenic primers), a forward and reverse mutagenic primers. The primers are constructed immediately upstream and downstream respectively of the codon of interest. The restriction and mutagenic primers are used to construct the first intermediate and second intermediate fragments. Two PCR reactions produce these linear intermediate fragments. Each of these linear intermediate fragments comprising at least one mutated codon of interest, a flanking nucleotide sequence and a digestion site. The third PCR reaction uses the two intermediate fragments and the forward and reverse restriction primers to produce a fused linear product. The opposite, here to for unattached ends of the linear product are digested with a restriction enzyme to create cohesive ends on the linear product. The cohesive ends of the linear product are fused by use of a DNA ligase to produce a circular product, e.g., a circular polynucleotide sequence.

To construct the intermediate fragments, the design and synthesis of two sets of forward and reverse primers are performed, a first set containing a restriction enzymes digestion site together with its flanking nucleotide sequence, and the second set contains at least one variant codon of interest (mutagenic primers). Those skilled in the art will recognize that the number of variants will depend upon the number of variant amino acid modifications desired. It is contemplated by the inventor that if other restriction enzymes are used in the process, the exact location of this digestion site and the corresponding sequence of the forward and reverse primers may be altered accordingly. In one embodiment, *Apa*I (4341) was selected as the digestion site. Fig. 2.

The term "primer" as used herein refers to an oligonucleotide whether occurring naturally as in a purified restriction digest or produced synthetically, which is capable of acting as a point of initiation of synthesis when placed under conditions in which synthesis of a primer extension product which is complementary to a nucleic acid strand is induced, i.e., in the presence of nucleotides and an agent for polymerization such as DNA polymerase and at a suitable temperature and pH. The primer is preferably single stranded for maximum efficiency in amplification, but may alternatively be double stranded. If double stranded, the primer is first treated to separate its strands before being used to prepare extension products. Preferably, the primer is an oligodeoxyribonucleotide. The primer must be sufficiently long to prime the synthesis of extension products in the presence of the agent for polymerization. The exact lengths of the primers will depend on many factors, including temperature and source of primer. For example, depending on the complexity of the target sequence, the oligonucleotide primer typically contains 7-40 or more nucleotides, although it may contain fewer or more nucleotides. Short primer molecules generally require cooler temperatures to form sufficiently stable hybrid complexes with template. The oligonucleotide primers of the invention may be prepared using any suitable method, such as, for example, the phosphotriester and phosphodiester methods described above, or automated embodiments thereof. In one such automated embodiment diethylphosphoramidites are used as starting materials and may be synthesized as described by Beaucage et al, Tetrahedron Letters (1981), 22:1859-1862. One method for synthesizing oligonucleotides on a modified solid support is described in U.S. Pat. No. 4,458,055. It is also possible to use a primer which has been isolated from a biological source (such as a restriction endonuclease digest).

The primers herein are selected to be "substantially" complementary to the different strands of each specific sequence to be amplified. This means that the primers must be sufficiently complementary to hybridize with their respective strands. Therefore, the primer sequence need not reflect the exact sequence of the template. For example, a non-complementary nucleotide fragment may be attached to the 5' end of the primer, with the remainder of the primer sequence being complementary to the strand. Typically, and preferably, however, the non-complementary nucleotides will be in the middle of the primer. Thus, non-complementary bases or longer sequences can be interspersed into the primer, provided that the primer sequence has sufficient complementarity with the sequence of the strand to be amplified to hybridize therewith and thereby form a template for synthesis of the extension product of the other primer.

The terms "mutagenic primer" or "mutagenic oligonucleotide" (used interchangeably herein) are intended to refer to oligonucleotide compositions which correspond to a portion of the template sequence and which are capable of hybridizing thereto. With respect to mutagenic primers, the primer will not precisely match the template nucleic acid, the mismatch or mismatches in the primer being used to introduce the desired mutation into the nucleic acid library. As used herein, "non-mutagenic primer" or "non-mutagenic oligonucleotide" refers to oligonucleotide compositions which will match precisely to the template nucleic acid. Primers may be designed so that for at least one region at which a mutagenic primer has been included, there is also non-mutagenic primer included in the oligonucleotide mixture. By adding a mixture of mutagenic primers and non-mutagenic primers corresponding to at least one of said mutagenic primers, it is possible to produce a resulting nucleic acid library in which a variety of combinatorial mutational patterns are presented. For example, if it is desired that some of the members of the mutant nucleic acid library retain their precursor sequence at certain positions while other members are mutant at such sites, the non-mutagenic primers provide the ability to obtain a specific level of non-mutant members within the nucleic acid library for a given residue. With respect to corresponding mutagenic and non-mutagenic primers, it is not necessary that the corresponding oligonucleotides be of identical length, but only that there is overlap in the region corresponding to the mutation to be added.

"Contiguous mutations" means mutations which are presented within the same oligonucleotide primer. For example, contiguous mutations may be adjacent or nearby each other, however, they will be introduced into the resulting mutant template nucleic acids by the same primer.

"Discontiguous mutations" means mutations which are presented in separate oligonucleotide primers. For example, discontiguous mutations will be introduced into the resulting mutant template nucleic acids by separately prepared oligonucleotide primers.

The primers can be generated by those of skill in the art. For example, all primers were ordered at Europrim-Invitrogen^{®} (Invitrogen, Carlsbad, USA)] (50 nmole scale, desalted). Optionally phosphorylated primers can be used for direct ligation of the fusion product (to bypass restriction digestion).

For generation of the mutagenic primers, different uses will involve different considerations. Thus it is contemplated by the inventor that generation of site-saturated libraries, site directed mutagenesis or error prone PCR involve different considerations.

For generation of the site saturated library construction, the forward and reverse mutagenic primer enclose the one to three desired mutations in the middle of the primer with 7-30 bases of correct sequence on both sides. However, it may be necessary to use primers that are either shorter than seven bases or longer than thirty bases to obtain the mutagenesis result desired. In one embodiment 10-25 bases of correct sequence on each side is used. In one embodiment, 15 bases of correct sequence on each side is used. These mutations, which cover the codon of interest, are randomly synthesized:
- 1^{st} base of the codon: A, C, G or T
- 2^{nd} base of the codon: A, C, G or T
- 3^{rd} base of the codon: C or G.

For generation of the site specific variant construction, the forward and reverse mutagenic primer enclose the one to three desired mutations in the middle of the primer with 7-30 bases of correct sequence on both sides (flanking sequences). In one embodiment 10-25 bases of correct sequence on each side is used. In one embodiment, 15 bases of correct sequence on each side is used. These mutations, which cover the codon of interest, are specific for the desired amino acid and are synthesized by design. In one embodiment, the mutagenic primers are derived from *Bacillus* protease codon, polynucleotide and/or amino acid sequences. In another embodiment the sequences are derived from those corresponding to *Bacillus* protease BPN' numbering. In another embodiment, the sequences are derived from wild-type protease found in *Bacillus lentus* or *Bacillus amyloliquefaciens.* Those skilled in the art will recognize that the methods utilized with respect to these proteases are also applicable to other *Bacillus* species, for example *Bacillus subtilis* and/or *Bacillus licheniformis.* Comparison of the subtilisin sequences is depicted in Fig. 3a-b. Those skilled in the art would recognize that other *Bacillus* wild type and/or mutated protease sequences are useful in generating the mutagenic primers described herein.

Having constructed the respective primers, two individual overlapping fragments are generated by PCR techniques. A first fragment is generated using the reverse restriction site primer, the forward mutagenic primer and the plasmid template. A second intermediate fragment is generated using the forward restriction site primer, the reverse mutagenic primer and the plasmid template (Fig. 2). Those skilled in the art will recognize that the appropriate DNA polymerase is used under the appropriate conditions according to the manufacturers' instructions, e.g., appropriate buffer, dNTP, ligase and/or polymerase. As a result, intermediate fragments are constructed which have about 30 bases of overlap around the codon of interest.

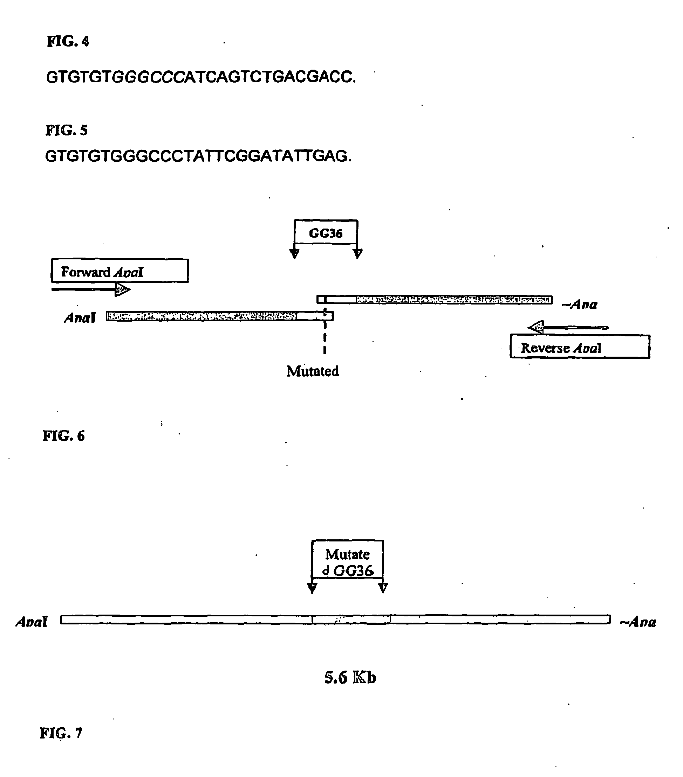

Having constructed two intermediate fragments, these fragments are fused to form a third, longer conjoined fragment. In one embodiment, the forward restriction site primer (Fig. 4), the reverse restriction site primer (Fig.5), and the intermediate fragments are used to generate the longer fragment, e.g. a full length linear product plasmid (Figs. 6 and 7). The longer fragment so produced may be purified at this time if desired by techniques known in the art.

Having constructed the longer fragment, cohesive ends are now produced thereon. In one embodiment, the selected restriction enzyme is contacted with the full-length fusion fragment. It is contemplated by the inventors that any appropriate buffers may be used. For example, it is recognized by those of skill in the art that some buffers may facilitate the enzymatic action of the restriction enzyme. For example, for *Apa*I, a 20mM Tris-HCI, 5 mM MgCl2, and 50 mM KCl buffer at pH 7.4 can be used.

An additional digestion with a second restriction enzyme can be performed on the resultant full length linear product plasmid with the cohesive ends. While not wanting to be bound by theory, the inventor believes that this may aid in reducing wild type background. In one embodiment, *Dpn*I can be used. This removes the plasmid template if desired. The full-length linear fragment with cohesive ends can be purified again.

A new plasmid is then constructed from the full-length linear fragment with cohesive ends. The fragment is contacted with a ligase in the appropriate medium to fuse the cohesive ends. Those skilled in the art will recognize that any ligase useful in the fusing of the cohesive ends can be used under the conditions and instructions provided by the manufacturers. In one embodiment, T4 DNA Ligase has shown usefulness.

General transformation procedures are taught in Current Protocols In Molecular Biology (vol. 1, edited by Ausubel et al., John Wiley & Sons, Inc. 1987, Chapter 9) and include calcium phosphate methods, transformation using DEAE-Dextran and electroporation. Plant transformation methods are taught in Rodriquez (WO 95/14099, published May 26, 1995).

In a preferred embodiment, the host is *Bacillus subtilis.* A circular product is introduced into a host cell via an expression vector capable of replicating within the *Bacillus* host cell. Suitable replicating plasmids for Bacillus are described in Molecular Biological Methods for Bacillus, Ed. Harwood and Cutting, John Wiley & Sons, 1990, see chapter 3 on plasmids. Suitable replicating plasmids for B. subtilis are listed on page 92. In one preferred embodiment, the pVS03 vector is used. In one preferred embodiment, the transformation of *Bacillus subtilis* is performed using the method of Anagnostopoulos and Spizizen (J. Bacteriol. 81, 741-746 (1961)) and selected for chloramphenicol resistance and protease activity as described more fully in the examples.

In vitro expression and screening methods may be used for selection and/or screening of the mutant template nucleic acids. Such methods are known in the art and are described in, for example, Hanes, J. and A. Pluckthun (1997) Proc. Natl. Acad. Sci. U S A 94, 4937-42.

As discussed in greater detail below, during the development of the present invention, site-saturation (NNS) libraries and site specific variants were constructed and directly transformed into *B. subtilis.* Enough DNA was produced and transformed to B.subtilis and resulted in sufficient levels of variant enzymes to enable comparisons with wild-type enzyme. These studies are described in detail in International Publications WO 03/062381 and WO 03/062380, both filed on January 16,2003. In addition, it was determined that a high expression protease plasmid that is toxic to *E. coli* could be directly transformed into *B. subtilis.* Thus, the present invention provides methods that are greatly improved over the standard methods used in the art.

### Definitions

Unless defined otherwise herein, all technical and scientific terms used herein have the same meaning as commonly understood by one of ordinary skill in the art to which this invention belongs *(See e.g.,* Singleton, et al., DICTIONARY OF MICROBIOLOGY AND MOLECULAR BIOLOGY, 2D ED., John Wiley and Sons, New York [1994]; and Hale & Marham, THE HARPER COLLINS DICTIONARY OF BIOLOGY, Harper Perennial, NY [1991], both of which provide one of skill with a general dictionary of many of the terms used herein). Although any methods and materials similar or equivalent to those described herein can be used in the practice or testing of the present invention, the preferred methods and materials are described. Numeric ranges are inclusive of the numbers defining the range. Unless otherwise indicated, nucleic acids are written left to right in 5' to 3' orientation; amino acid sequences are written left to right in amino to carboxy orientation, respectively. The headings provided herein are not limitations of the various aspects or embodiments of the invention that can be had by reference to the specification as a whole. Accordingly, the terms defined immediately below are more fully defined by reference to the specification as a whole.

As used herein, the term *"Apa*I*"* refers to the restriction site located at 4341 in the pVS08 5568 nucleic acid base pair *Bacillus subtilis* expression vector

As used herein, the term "partially overlapping" refers to polynucleotide sequences which share identical or complementary sequences which enable ligation of the separate sequences into a conjoined unitary sequence.

As used herein, the term "mutated codon of interest" refers to a mutant or modified codon encoding for a mutant amino acid residue.

As used herein, the term "digestion site primer" refers to a primer comprising a polynucleotide sequence wherein said digestion site is cleaved by a restriction enzyme.

As used herein, the term "forward primer" refers to a primer encoded in a first direction, e.g., the 5' to 3' direction or alternatively in the 3' to 5' direction depending upon the direction of the reverse primer

As used herein, the term "mutagenic primer" refers to a primer comprising a mutated codon of interest.

As used herein, the term "reverse primer" refers to a primer encoded in the opposite direction of the forward primer, e.g., 3' to 5' direction or alternatively the 5' to 3' direction, depending upon the direction of the forward primer.

As used herein, the term "linear" refers to a nucleotide or codon segment having opposite ends not joined to each other.

As used herein, the term "fusion PCR" refers to PCR methodology which is used to join or fuse a plurality of polynucleotide fragments into a conjoined polynucleotide fragment.

As used herein, the term "digestion site" refers to the nucleotide segment which the particular restriction enzyme cleaves.

As used herein, "host cell" refers to a cell that has the capacity to act as a host and expression vehicle for an incoming sequence. In the present invention, host cells are members of the genus *Bacillus.* As used herein, the genus *Bacillus* includes all of the species known to those of skill in the art, including but not limited to *B. subtilis, B. licheniformis, B. lentus, B. brevis, B. stearothermophilus, B. alcalophilus, B. amyloliquefaciens, B. coagulans, B. circulans, B. lautus, B.clausii,* and *B. thuringiensis.*

As used herein, the terms "DNA construct" and "transforming DNA" are used interchangeably to refer to DNA used to introduce sequences into a host cell or organism. The DNA is generated *in vitro* by PCR. In particularly preferred embodiments, the DNA construct comprises a sequence of interest (e.g., as an incoming sequence). In some embodiments, the sequence is operably linked to additional elements such as control elements (e.g., promoters, etc.). The DNA construct may further comprise a selectable marker. It may further comprise an incoming sequence flanked by homology boxes. In a further embodiment, the transforming DNA comprises other non-homologous sequences, added to the ends (e.g., stuffer sequences or flanks). In some embodiments, the ends of the incoming sequence are closed such that the transforming DNA forms a closed circle. The transforming sequences may be wild-type, mutant or modified. In some embodiments, the DNA construct comprises sequences homologous to the host cell chromosome. In other embodiments, the DNA construct comprises non-homologous sequences. Once the DNA construct is assembled *in vitro* it may be used to: 1) insert heterologous sequences into a desired target sequence of a host cell, and/or 2) mutagenize a region of the host cell chromosome (i.e., replace an endogenous sequence with a heterologous sequence), 3) delete target genes; and/or introduce a replicating plasmid into the host.

The present invention involves assembling a DNA construct in *vitro,* followed by direct cloning of such construct into a competent *Bacillus.* PCR fusion and ligation can be employed to assemble a DNA construct *in vitro.* The DNA construct comprises a DNA into which a mutation has been introduced. In alternative embodiments, highly competent mutants of *Bacillus* are preferably employed to facilitate the direct cloning of the constructs into the cells. For example, *Bacillus* carrying the comK gene under the control of a xylose-inducible promoter (Pxyl-comK) can be reliably transformed with very high efficiency *(See e.g.,* Hahn et al., Mol. Microbiol., 21:763-775 [1996]).

As used herein, the term "direct transformation" means that an intermediate cell is not used to amplify or otherwise process the DNA construct prior to introduction into the host cell. Introduction of the DNA construct into the host cell includes those physical and chemical methods known in the art to introduce DNA into a host cell. Such methods include but are not limited to calcium chloride precipitation, electroporation, naked DNA, liposomes, and the active uptake of DNA by a competent host, etc. In some embodiments, a library of mutants is generated.

As used herein, the term "targeted randomization" refers to a process that produces a plurality of sequences where one or several positions have been randomized. In some embodiments, randomization is complete (*i.e*., all four nucleotides, A, T, G, and C can occur at a randomized position. In alternative embodiments, randomization of a nucleotide is limited to a subset of the four nucleotides. Targeted randomization can be applied to one or several codons of a sequence, coding for one or several proteins of interest. When expressed, the resulting libraries produce protein populations in which one or more amino acid positions can contain a mixture of all 20 amino acids or a subset of amino acids, as determined by the randomization scheme of the randomized codon. In some embodiments, the individual members of a population resulting from targeted randomization differ in the number of amino acids, due to targeted or random insertion or deletion of codons. In further embodiments, synthetic amino acids are included in the protein populations produced.

In some preferred embodiments, mutant DNA sequences are generated with site saturation mutagenesis in at least one codon. In other preferred embodiments, site saturation mutagenesis is performed for two or more codons. In a further embodiment, mutant DNA sequences have more than 40%, more than 45%, more than 50%, more than 55%, more than 60%, more than 65%, more than 70%, more than 75%, more than 80%, more than 85%, more than 90%, more than 95%, or more than 98% homology with the wild-type sequence. Alternatively, mutant DNA may be generated *in vivo* using any known mutagenic procedure (e.g., radiation, nitrosoguanidine, etc.). The DNA construct sequences may be wild-type, mutant or modified. In addition, the sequences may be homologous or heterologous.

An "incoming sequence" as used herein means a DNA sequence that is newly introduced into the host cell. In some embodiments, the incoming sequence becomes integrated into the host chromosome or genome. The sequence may encode one or more proteins of interest. Thus, as used herein, the term "sequence of interest" refers to an incoming sequence or a sequence to be generated by the host cell. The terms "gene of interest" and "sequence of interest" are used interchangeably herein.

The incoming sequence may comprise a promoter operably linked to a sequence of interest. An incoming sequence comprises a sequence that may or may not already present in the genome of the cell to be transformed (*i.e.,* homologous and heterologous sequences find use with the present invention).

In one embodiment, the incoming sequence encodes at least one heterologous protein, including, but not limited to hormones, enzymes, and growth factors. In an alternative embodiment, the incoming sequence encodes a functional wild-type gene or operon, a functional mutant gene or operon, or a non-functional gene or operon. In some embodiments, the non-functional sequence is inserted into a target sequence to disrupt function, thereby allowing a determination of function of the disrupted gene.

The terms "wild-type sequence," or "wild-type gene" are used interchangeably herein, to refer to a sequence that is native or naturally occurring in a host cell. In some embodiments, the wild-type sequence refers to a sequence of interest that is the starting point of a protein engineering project. The wild-type sequence may encode either a homologous or heterologous protein. A homologous protein is one the host cell would produce without intervention. A heterologous protein is one that the host cell would not produce but for the intervention.

The terms "modified sequence" and "modified genes" are used interchangeably herein to refer to a sequence that includes a deletion, insertion or interruption of naturally occurring nucleic acid sequence. In some preferred embodiments, the expression product of the modified sequence is a truncated protein (*e.g.,* if the modification is a deletion or interruption of the sequence). In some particularly preferred embodiments, the truncated protein retains biological activity. In alternative embodiments, the expression product of the modified sequence is an elongated protein (e.g., modifications comprising an insertion into the nucleic acid sequence). In some embodiments, an insertion leads to a truncated protein (e.g., when the insertion results in the formation of a stop codon). Thus, an insertion may result in either a truncated protein or an elongated protein as an expression product.

As used herein, the terms "mutant sequence" and "mutant gene" are used interchangeably and refer to a sequence that has an alteration in at least one codon occurring in a host cell's wild-type sequence. The expression product of the mutant sequence is a protein with an altered amino acid sequence relative to the wild-type. The expression product may have an altered functional capacity (e.g., enhanced enzymatic activity).

As used herein, a "flanking sequence" refers to any sequence that is either upstream or downstream of the sequence being discussed (e.g., for genes A B C, gene B is flanked by the A and C gene sequences). In a preferred embodiment, the incoming sequence is flanked by a homology box on each side. In another embodiment, the incoming sequence and the homology boxes comprise a unit that is flanked by stuffer sequence on each side. In some embodiments, a flanking sequence is present on only a single side (either 3' or 5'), but in preferred embodiments, it is on each side of the sequence being flanked.

As used herein, the term "stuffer sequence" refers to any extra DNA that flanks homology boxes (typically vector sequences). However, the term encompasses any non-homologous DNA sequence. Not to be limited by any theory, a stuffer sequence provides a noncritical target for a cell to initiate DNA uptake.

As used herein, the term "homologous sequence" refers to a sequence that is found in the same genetic source or species. For example, the host cell strain may be deficient in a specific gene. If that gene is found in other strains of the same species the gene would be considered a homologous sequence.

As used herein, the term "heterologous sequence" refers to a sequence derived from a separate genetic source or species. Heterologous sequences encompass non-host sequences, modified sequences, sequences from a different host cell strain, and homologous sequences from a different chromosomal location of the host cell. In some embodiments, homology boxes flank each side of an incoming sequence

As used herein, the term "chromosomal integration" refers to the process whereby the incoming sequence is introduced into the chromosome of a host cell (*e.g., Bacillus*). The homology boxes of the transforming DNA align with homologous regions of the chromosome. Subsequently, the sequence between the homology boxes is replaced by the incoming sequence in a double crossover (i.e., homologous recombination).

As used herein, the term "target sequence" refers to a DNA sequence in the host cell that encodes the sequence where it is desired for the incoming sequence to be inserted into the host cell genome. In some embodiments, the target sequence encodes a functional wild-type gene or operon, while in other embodiments the target sequence encodes a functional mutant gene or operon, or a non-functional gene or operon.

As used herein, the term "selectable marker" refers to genes that provide an indication that a host cell has taken up an incoming DNA of interest or some other reaction has occurred. Typically, selectable markers are genes that confer antibiotic resistance or a metabolic advantage on the host cell to allow cells containing the exogenous DNA to be distinguished from cells that have not received any exogenous sequence during the transformation. A "residing selectable marker" is one that is located on the chromosome of the microorganism to be transformed. A residing selectable marker encodes a gene that is different from the selectable marker on the transforming DNA construct.

As used herein, the term "library of mutants" refers to a population of cells which are identical in most of their genome but include different homologues of one or more genes. Such libraries can be used, for example, to identify genes or operons with improved traits.

As used herein, the terms "hyper competent" and "super competent" mean that greater than 1% of a cell population is transformable with chromosomal DNA *(e.g., Bacillus* DNA). Alternatively, the terms are used in reference to cell populations in which greater than10% of a cell population is transformable with a self-replicating plasmid *(e.g., a Bacillus* plasmid). Preferably, the super competent cells are transformed at a rate greater than observed for the wild-type or parental cell population. Super competent and hyper competent are used interchangeably herein.

### EXPERIMENTAL

The following examples are provided in order to demonstrate and further illustrate certain preferred embodiments and aspects of the present invention and are not to be construed as limiting the scope thereof.

In the experimental disclosure which follows, the following abbreviations apply: °C (degrees Centigrade); rpm (rotations per minute); H₂O (water); dH₂O (deionized water); (HCl (hydrochloric acid); aa (amino acid); bp (base pair); kb (kilobase pair); kD (kilodaltons); gm (grams); µg (micrograms); mg (milligrams); ng (nanograms); µl(microliters); ml (milliliters); mm (millimeters); nm (nanometers); µm (micrometer); M (molar); mM (millimolar); µM(micromolar); U (units); V (volts); dNTP (deoxynucleoside triphosphates); MOPS (3-(*N-*morpholino)propanesulfonic acid); MW (molecular weight); sec (seconds); min(s) (minute/minutes); hr(s) (hour/hours); CuCl₂ (cupric chloride); CoCl₂ (cobalt chloride); FeSO₄ (ferrous sulfate); KCl (potassium chloride); K₂HPO₄ (Potassium Phosphate, dibasic); KH₂PO₄ (potassium phosphate, monobasic); K₂SO₄ (potassium sulfate); KOH (potassium hydroxide); MgCl₂ (magnesium chloride); MgSO₄ (magnesium sulfate); MnSO₄ (manganese sulfate); NaCl (sodium chloride); NaMoO₄ (sodium molybdate); NaB₄O₇ (sodium borate); Na₃Citrate (sodium citrate); Maltrin 150 (maltodextrin); OD₅₇₅ (optical density at 575 nm); (NH₄)₂SO₄ (ammonium sulfate); PAGE (polyacrylamide gel electrophoresis); PBS (phosphate buffered saline [150 mM NaCl, 10 mM sodium phosphate buffer, pH 7.2]); PEG (polyethylene glycol); PCR (polymerase chain reaction); RT-PCR (reverse transcription PCR); SDS (sodium dodecyl sulfate); Tris (tris(hydroxymethyl)aminomethane); w/v (weight to volume); v/v (volume to volume); LA medium (per liter: Difco Tryptone Peptone 20g, Difco Yeast Extract 10g, EM Science NaCl 1g, EM Science Agar 17.5g, dH20 to 1L); TSB (tryptic soya broth, tryptone soy broth); Tris-HCl (2-amino-2(hydroxymethyl)-1,3-propanediol hydrochloride or Tris (hydroxymethyl)aminomethane); Tris-SO₄ (Tris sulfate); ATCC (American Type Culture Collection, Rockville, MD); Difco (Difco Laboratories, Detroit, MI); Europrim-Invitrogen (Invitrogen Corporation, Carlsbad, CA USA); GIBCO BRL or Gibco BRL (Life Technologies, Inc., Gaithersburg, MD); Invitrogen (Invitrogen Corp., Carlsbad, CA); MJ Research (MJ Research, Inc., Waltham, MA); Sigma (Sigma Chemical Co., St. Louis, MO); Roche (Hoffmann-La Roche, Basel, Switzerland); EM Science (EM Science, Gibbstown, NJ); and Qiagen (Qiagen, Inc., Valencia, CA).

### EXAMPLE 1

### SITE-DIRECTED MUTAGENESIS WITH FORWARD AND REVERSE 5' PHOSPHORYLATED PRIMERS

In this Example, various experiments conducted for direct *Bacillus* transformation are described.

A large number of protease variants were produced and purified using methods well known in the art. All mutations were made in *Bacillus lentus* GG36 subtilisin protease (Fig. 3A-B, SEQ ID NO.:1 (US Patent No. 6,482,628; International Publication WO 99/20769, published April 29, 1999). Some of the variants were made as described in International Publication WO 03/062381, filed January 16, 2003, and International Publication WO 03/06280, filed January 16, 2003.

This example was to incorporate random mutations at a specific GG36 codon. The GG36 gene was located in the pVS08 *B.subtilis* expression vector.

### Example 1A Construction of circular DNA (Fig.1)

To construct the GG36 site saturated libraries and site specific variants, three PCR reactions were performed: two PCR's to introduce the mutated codon of interest in GG36 and a fusion PCR to construct the expression vector including the desired mutation(s). The GG36 codons of interest were numbered according to the BPN' numbering

### For the site saturated library construction:

The method of mutagenesis was based on the region-specific mutation approach in which the creation of all possible mutations at a time in a specific DNA codon was performed using a forward and reversed complimentary oligonucleotide primer set with a length of 30 up to 40nucleotides enclosing a specific designed triple DNA sequence NNS ((A,C,T or G), (A,C,T or G), (C or G)) that corresponded with the sequence of the codon to be mutated and guaranteed randomly incorporation of nucleotides at that codon.

### For the Site specific variant construction:

The forward and reverse mutagenic primer enclosed the desired mutation(s) in the middle of the primer with ~15 bases of homologues sequence on both sides. These mutation(s), which cover the codon of interest, are specific for the desired amino acid and were synthesized by design.

The second primer set used to construct the libraries and variants contained the pVS08 *Apa*I digestion site together with its flanking nucleotide sequence (e.g., with 27 additional nucleotides). Primers were produced by Europrim-Invitrogen (50nmole scale, desalted).

### ApaI primers :

Forward *Apal* primer:
GTGTGT*GGGCCC*ATCAGTCTGACGACC

Reverse *Apal* primer:
GTGTGTGGGCCCTATTCGGATATTGAG

The introduction of the mutation(s) in GG36 molecules was performed using Invitrogen (Carlsbad, CA, USA) Platinum^{®} *Taq* DNA Polymerase High Fidelity (Cat. no. 11304-102) together with pVS08 template DNA and Forward mutagenic primer and Reverse *Apa*I primer for reaction 1, or Reverse mutagenic primer and Forward *Apa*I primer for reaction 2.

The construction of the expression vector including the desired mutation(s) was accomplished by a fusion PCR using PCR fragment of both reaction 1 and 2, forward and reverse *Apa*I primer and Invitrogen Platinum^{®} *Taq* DNA Polymerase High Fidelity (Cat. no. 11304-102). All PCR's were executed according to Invitrogen protocol supplied with the polymerases, except for the number of cycles: 20 instead of 30. Two separate PCR reactions are performed using Invitrogen Platinum^{®} *Tag* DNA Polymerase High Fidelity (Cat. no. 11304-102): The PCR programs for both mixes were :

| | |
|---|---|
| 2 min. | 95° C |
| 30 sec | 94° C |
| 30 sec | 55° C |
| 3:20 min | 68° C |
| 7 min | 68°C |

using a MJ Research (Location) PTC-200 Peltier thermal cycler (20 cycli). The PCR experiments resulted in two approximately 2.8 Kb fragments which had about 30 nucleotide base overlap around the *Bacillus* codon of interest. Fragments were fused in a third PCR reaction using these two aforementioned fragments and the forward and reverse *ApaI* primers (SEQ ID Nos. 1 and 2, Figs. 3 and 4, primer sequence data listed on page 13). The fusion PCR reaction was carried out in the following solution:
The amplified linear 5.6 Kb fragment was purified (using Qiagen^{®} Qiaquick PCR purification kit Cat. no. 28106) and digested with *Apa*I restriction enzyme to create cohesive ends on both sides of the fusion fragment:
   - 35 µL purified DNA fragment
   - 4 µL React^{®} 4 buffer (Invitrogen^{®}: 20 mM Tris-HCl, 5 mM MgCl₂, 50 mM KCl, pH 7.4)
   - 1 µL *Apa*I, 10 units/ml (Invitrogen^{®} Cat. no. 15440-019)
   Reaction conditions: 1 hour, 30°C.
   Optionally:

An additional digestion with Invitrogen *Dpn*I was performed to remove the pVS08 template DNA:
   - 40 µL *Apa*I digested DNA fragment
   - 1 µL *Dpn*I, 4 units/µL (Invitrogen^{®} Cat. no. 15242-019)
Reaction conditions: 16-20 hours, 37°C.

Ligation of the double digested and purified fragment results in new circular DNA containing the desired mutation with was directly transformed to competent *Bacillus subtilis* :
- 30 µL of purified *Apa*I (and *Dpn*I) digested DNA fragment
- 8 µL T4 DNA Ligase buffer (Invitrogen^{®} Cat. no. 46300-018)
- 1 µL T4 DNA Ligase, 1 uilit/µL (Invitrogen^{®} Cat. no. 15224-017)
Reaction conditions: 16-20 hours, 16°C.

### Example 1b Transformation of Bacillus subtilis

Ligation mixtures were transformed to *Bacillus subtilis* BG2864 (Naki *et al.,* 1998) using the method of Anagnostopoulos and Spizizen (1961) and selected for chloramphenicol resistance and protease activity.

### Materials

### 2x Spizizen medium

per liter:
28 g K₂HPO₄
12 g KH₂PO₄
4 g (NH₄)₂SO₄
2 g tri-Sodium citrate (C₆H₅Na₃O₇)
0.4 g MgSO₄.7H₂O
pH 7.0 - 7.4

### 2x Spizizen-plus medium

Added 1 ml 50% Glucose and 100µl 20% Bacto^{®} Casamino acids solution (Difco Cat. no. 0230-15) to 100 ml 2x Spizizen medium.

### HI-agar

Difco Bacto^{®} Heart infusion agar (Cat. no. 0044-17)
Suspended 40 g/L in deionized water.
Autoclaved at 121°C for 15 minutes

### Minimal medium Agar:

Solution A: per liter
10 g K₂HPO₄
6 g KH₂PO₄
2 g (NH₄)₂SO₄
1 g tri-Sodium citrate (C₆H₅Na₃O₇.2H₂O)
0.2 g MgSO₄.7H₂O
250 ug MnSO₄.4H₂O
2 g L-Glutanuc acid

Solution B: per liter
35 g Difco Bacto^{®} agar (Cat. no. 0140-15)

Solution C:
Sterilized solution A and B, cooled down to 50°C and mixed equal volumes.
Added per liter:
   10 ml 50% glucose
   1 ml 20% Casamino acids solution
   100 ml 4% Casein
   Antibiotic - 5 mg/Liter Chloramphenicol

### Method

Day 1: *Bacillus subtilis* (source) was inoculated on a HI-agar plate and incubated overnight at 37°C.

Day 2: During the morning: Added a fresh colony of *Bacillus subtilis* from the HI agar plate into a 500 ml shake flask containing 10 ml 2x Spizizen-plus medium. This fresh colony was incubated overnight in a 37°C water bath by gently shaking (not orbital), ± 50 shakes per minute (pm).

Day 3: 90 ml 37°C pre-warmed 2x Spizizen-plus medium was added to the shake flask, incubated at 37°C/220 rpm. When O.D.₅₇₅ ≈ 1.0, 100 ml 37°C pre-warmed 2x Spizizen medium was added to the flask and the flask incubated for 1½ hour at 37°C/220 rpm and the resulting *Bacillus* cells were ready for transformation.

39 µL ligated DNA mix of interest were then added to 1 ml of transformation ready (competent) Bacillus cells and the resulting transformation mixture was incubated in small flasks for 1 hour at 37°C/220 rpm. The cells were then spread cells on minimal medium agar plates. The plates were left to dry (standing at room temperature) to dry for 30 minutes and incubated overnight at 37°C.

Day 4: Transformed *Bacillus sublilis* colonies were selected for chloramphenicol resistance and protease activity on skim milk plates, inoculated in TSB medium containing 5 mg/Liter Chloramphenicol and 10% glycerol and incubated overnight at 37°C/220 rpm
Day 5: Glycerol containing cultures are directly stored at -80°C.

### Example 1c

### Incubate of Bacillus subtilis transformants for protein production

### Materials

### MOPS medium

According to: Culture Medium for Enterobacteria by Frederick C. Neidhardt, Philip L. Bloch and David F. Smith in Journal of Bacteriology, Sept 1974. p736-747 Vol. 119. No. 3

### Example 1d

### Method for protein production

5 µL of glycerol culture (-80°C store) from Example 1b was inoculated in micro titer plate or shake flask with MOPS medium [200 µl up to 25 ml]. The resulting culture was incubated for 3 days at 37°C/220 rpm

### Example 1e

### Method for protein production

In another example, 1-50 µL of glycerol culture was inoculated in MOPS media (Frederick C. Neidhardt *et al.,* 1974) containing carbon source (Glucose and Maltodextrine, 10.5 and 17.5 g/l) a nitrogen source (Urea, 3.6 g/l), and essential nutrients such as phosphate (0.5 g/l) and sulphate (0.5 g/l) and further supplemented with trace elements (Fe, Mn, Zn, Cu, Co, 1-4 mg/ml). The medium was buffered with a MOPS/Tricine mixture resulting in a pH varying 7 to 8. The culture was incubated for 1-5 days at 37°C/220 rpm.

### References:

Selection of a subtilisin-hyperproducing Bacillus in a highly structured environment by D. Naki, C. Paech, G. Ganshaw, V. Schellenberger. Appl Microbiol Biotechnol (1998) 49:290-294.
Requirements for transformation in Bacillus subtilis by Anagnostopoulos, C. and Spizizen, J. in J. Bacteriol. 81, 741-746 (1961).
Culture Medium for Enterobacteria by Frederick C. Neidhardt, Philip L. Bloch and David F. Smith in Journal of Bacteriology, Sept 1974. p736-747 Vol. 119. No. 3.

### Results

Sufficient enzyme was produced by this methodology to enable comparison of variant enzyme characteristics with that of the wild-type.

## Claims

1. A method for direct transformation of a Bacillus host cell comprising the steps:
(a) generating partially overlapping intermediate fragments by polymerase chain reaction, said partially overlapping intermediate fragments further comprising a first intermediate fragment and a second intermediate fragment,
wherein said first intermediate fragment is generated by polymerase chain reaction using a forward mutagenic primer and a reverse digestion site primer, and a plasmid template which is an expression vector capable of replicating within the Bacillus host cell, and
wherein said second intermediate fragment is generated by polymerase chain reaction using a reverse mutagenic primer and a forward digestion site primer and the plasmid template,
such that said first and second intermediate fragments each comprise at least one mutated codon of interest, a flanking nucleotide sequence and a digestion site
(b) joining ends of said intermediate fragments to produce a linear product by fusion polymerase chain reaction;
(c) digesting the linear product with a restriction enzyme to create cohesive ends on the linear product;
(d) ligating the cohesive ends of the linear product to create a circular product which is an expression vector capable of replicating within the Bacillus host cell; and
(e) incubating said Bacillus host cell with said circular product.

2. The method of claim 1 wherein said digestion site is an *Apal* digestion site and said restriction enzyme is *Apal.*

3. The method of claim 2 wherein said forward digestion site primers comprises the polynucleotide sequence GTGTGTGGGCCCATCAGTCTCACGACC.

4. The method of claim 2 wherein said reverse digestion site primers comprises the polynucleotide sequence GTGTGTGGGCCCTATTCGGATATTGAG.

5. A method for constructing a vector for direct transformation of a Bacillus host cell, the method comprising the steps:
(a) generating partially overlapping intermediate fragments by polymerase chain reaction, said partially overlapping intermediate fragments further comprising a first intermediate fragment and a second intermediate fragment,
wherein said first intermediate fragment is generated by polymerase chain reaction using a forward mutagenic primer and a reverse digestion site primer, and a plasmid template which is an expression vector capable of replicating within the Bacillus host cell, and
wherein said second intermediate fragment is generated by polymerase chain reaction using a reverse mutagenic primer and a forward digestion site primer and the plasmid template,
and wherein said forward and reverse mutagenic primers have an overlapping portion upstream and downstream of a codon of interest,
such that said first and second intermediate fragments each comprise at least one mutated codon of interest, a flanking nucleotide sequence and a digestion site;
(b) joining ends of said intermediate fragments to produce a linear product by fusion polymerase chain reaction;
(c) digesting the linear product with a restriction enzyme to create cohesive ends on the linear product; and
(d) ligating of the cohesive ends of the linear product to create a circular polynucleotide sequence which is an expression vector capable of replicating within the Bacillus host cell.

6. The method of claim 5 wherein said forward digestion site primer comprises the polynucleotide sequence GTGTGTGGGCCCATCAGTCTCACGACC.

7. The method of claim 5 wherein said reverse digestion site primer comprises the polynucleotide sequence GTGTGTGGGCCCTATTCGGATATTGAG.

## Patentansprüche

1. Verfahren zur direkten Transformation einer Bacillus-Wirtszelle, welches die folgenden Schritte umfasst:
(a) Erzeugen von partiell überlappenden Zwischenfragmenten durch die Polymerasekettenreaktion, wobei die partiell überlappenden Zwischenfragmente ferner ein erstes Zwischenfragment und ein zweites Zwischenfragment umfassen,
wobei das erste Zwischenfragment durch die Polymerasekettenreaktion unter Verwendung eines mutagenen Vortwärts-Primers und eines Rückwärts-Verdauungsstellen-Primers und einer Plasmidmatrize, bei der es sich um einen Expressionsvektor handelt, der in der Lage ist, sich in der Bacillus-Wirtszelle zu replizieren, erzeugt wird und
wobei das zweite Zwischenfragment durch die Polymerasekettenreaktion unter Verwendung eines mutagenen Rückwärts-Primers und eines Vorwärts-Verdauungsstellen-Primers und der Plasmidmatrize erzeugt wird,
so dass das erste und das zweite Zwischenfragment jeweils mindestens ein mutiertes Codon von Interesse, eine flankierende Nukleotidsequenz und eine Verdauungsstelle umfassen;
(b) Verknüpfen der Enden der Zwischenfragmente zur Herstellung eines linearen Produkts durch Fusions-Polymerasekettenreaktion;
(c) Verdauen des linearen Produkts mit einem Restriktionsenzym zur Erzeugung kohäsiver Enden auf dem linearen Produkt;
(d) Ligieren der kohäsiven Enden des linearen Produkts zur Erzeugung eines kreisförmigen Produkts, bei dem es sich um einen Expressionsvektor handelt, der in der Lage ist, sich in der Bacillus-Wirtszelle zu replizieren, und
(e) Inkubieren der Bacillus-Wirtszelle mit dem kreisförmigen Produkt.

2. Verfahren nach Anspruch 1, worin die Verdauungsstelle eine Apal-Verdauungsstelle ist und das Restriktionsenzym Apal ist.

3. Verfahren nach Anspruch 2, worin der Vorwärts-Verdauungsstellen-Primer die Polynukleotidsequenz GTGTGTGGGCCCATCAGTCTCACGACC umfasst.

4. Verfahren nach Anspruch 2, worin der Rückwärts-Verdauungsstellen-Primer die Polynukleotidsequenz GTGTGTGGGCCCTATTCGGATATTGAG umfasst.

5. Verfahren zur Konstruktion eines Vektors zur direkten Transformation einer Bacillus-Wirtszelle, wobei das Verfahren die folgenden Schritte umfasst:
(a) Erzeugen von partiell überlappenden Zwischenfragmenten durch die Polymerasekettenreaktion, wobei die partiell überlappenden Zwischenfragmente ferner ein erstes Zwischenfragment und ein zweites Zwischenfragment umfassen,
wobei das erste Zwischenfragment durch die Polymerasekettenreaktion unter Verwendung eines mutagenen Vorwärts-Primers und eines Rückwärts-Verdauungsstellen-Primers und einer Plasmidmatrize, bei der es sich um einen Expressionsvektor handelt, der in der Lage ist, sich in der Bacillus-Wirtszelle zu replizieren, erzeugt wird und
wobei das zweite Zwischenfragment durch die Polymerasekettenreaktion unter Verwendung eines mutagenen Rückwärts-Primers und eines Vorwärts-Verdauungsstellen-Primers und der Plasmidmatrize erzeugt wird,
und wobei der mutagene Vorwärts- und der mutagene Rückwärts-Primer jeweils einen überlappenden Abschnitt stromauf und stromab von einem Codon von Interesse aufweisen,
so dass das erste und das zweite Zwischenfragment jeweils mindestens ein mutiertes Codon von Interesse, eine flankierende Nukleotidsequenz und eine Verdauungsstelle umfassen;
(b) Verknüpfen der Enden der Zwischenfragmente zur Herstellung eines linearen Produkts durch Fusions-Polymerasekettenreaktion;
(c) Verdauen des linearen Produkts mit einem Restriktionsenzym zur Erzeugung kohäsiver Enden auf dem linearen Produkt;
(d) Ligieren der kohäsiven Enden des linearen Produkts zur Erzeugung einer kreisförmigen Polynukleotidsequenz, bei der es sich um einen Expressionsvektor handelt, der in der Lage ist, sich in der Bacillus-Wirtszelle zu replizieren.

6. Verfahren nach Anspruch 5, worin der Vorwärts-Verdauungsstellen-Primer die Polynukleotidsequenz GTGTGTGGGCCCATCAGTCTCACGACC umfasst.

7. Verfahren nach Anspruch 5, worin der Rückwärts-Verdauungsstellen-Primer die Polynukleotidsequenz GTGTGTGGGCCCTATTCGGATATTGAG umfasst.

## Revendications

1. Procédé de transformation directe d'une cellule hôte Bacillus comprenant les étapes suivantes:
(a) production de fragments intermédiaires partiellement chevauchants par amplification en chaîne par polymérase, lesdits fragments intermédiaires partiellement chevauchants comprenant en outre un premier fragment intermédiaire et un second fragment intermédiaire,
où ledit premier fragment intermédiaire est produit par amplification en chaîne par polymérase en utilisant une amorce mutagène directe et une amorce de site de digestion µinverse, et une matrice plasmidique qui est un vecteur d'expression apte à répliquer dans la cellule hôte *Bacillus,* et
où ledit deuxième fragment intermédiaire est produit par amplification en chaîne par polymérase en utilisant une amorce mutagène inverse et une amorce de site de digestion directe et la matrice plasmidique,
de telle sorte que lesdits premier et second fragments intermédiaires comprennent chacun au moins un codon muté d'intérêt, une séquence nucléotidique adjacente et un site de digestion;
(b) réunion des extrémités desdits fragments intermédiaires pour produire un produit linéaire par amplification en chaîne par polymérase par fusion;
(c) digestion du produit linéaire avec une enzyme de restriction pour créer des extrémités cohésives sur le produit linéaire;
(d) ligature des extrémités cohésives du produit linéaire pour créer un produit circulaire qui est un vecteur d'expression apte à répliquer dans la cellule hôte *Bacillus;* et
(e) incubation de ladite cellule hôte de *Bacillus* avec ledit produit circulaire.

2. Procédé selon la revendication 1, dans lequel ledit site de digestion est un site de digestion *Apal*, et ladite enzyme de restriction est *Apal.*

3. Procédé selon la revendication 2, dans lequel ladite amorce directe du site de digestion comprend la séquence polynucléotidique GTGTGTGGGCCCATCAGTCTCACGACC.

4. Procédé selon la revendication 2, dans lequel ladite amorce inverse du site de digestion comprend la séquence polynucléotidique GTGTGTGGGCCCTATrCGGATATTGAG.

5. Procédé pour construire un vecteur pour la transformation directe d'une cellule hôte de *Bacillus,* le procédé comprenant les étapes:
(a) production de fragments intermédiaires partiellement chevauchants par amplification en chaîne par polymérase, lesdits fragments intermédiaires partiellement chevauchants comprenant en outre un premier fragment intermédiaire et un second fragment intermédiaire,
où ledit premier fragment intermédiaire est produit par amplification en chaîne par polymérase en utilisant une amorce mutagène directe et une amorce inverse du site de digestion, et une matrice plasmidique qui est un vecteur d'expression apte à répliquer dans la cellule hôte de *Bacillus,* et
où ledit second fragment intermédiaire est produit par amplification en chaîne par polymérase en utilisant une amorce mutagène inverse et une amorce directe du site de digestion et la matrice plasmidique,
et où lesdites amorces mutagènes directe et inverse ont une portion chevauchante en amont et en aval d'un codon d'intérêt,
de telle sorte que lesdits premier et second fragments intermédiaires comprennent chacun au moins un codon muté d'intérêt, une séquence nucléotidique adjacente et un site de digestion;
(b) réunion des extrémités desdits fragments intermédiaires pour produire un produit linéaire par amplification en chaîne par polymérase par fusion;
(c) digestion du produit linéaire avec une enzyme de restriction pour créer des extrémités cohésives sur le produit linéaire; et
(d) ligature des extrémités cohésives du produit linéaire pour créer une séquence de polynucléotides circulaires qui est un vecteur d'expression apte à répliquer dans la cellule hôte de *Bacillus.*

6. Procédé selon la revendication 5, dans lequel ladite amorce directe du site de digestion comprend la séquence polynucléotidique GTGTGTGGGCCCATCAGTCTCACGACC.

7. Procédé selon la revendication 5, dans lequel ladite amorce inverse du site de digestion comprend la séquence polynucléotidique GTGTGTGGGCCCTATTCGGATATTGAG.
